# EUROPÄISCHE PATENTSCHRIFT

(11) **EP 1 567 215 B1**
(45) Veröffentlichungstag und Bekanntmachung des Hinweises auf die Patenterteilung: **06.01.2010**
(21) Anmeldenummer: 03811708.1
(22) Anmeldetag: 17.11.2003
(51) Int. Cl.: A61M 5/32, A61M 5/34, A61M 5/00, B25B 23/14

(54) **VORRICHTUNG ZUM VERSCHRAUBEN MEDIUMFUEHRENDER GEWINDEVERBINDUNGEN, INSBESONDERE LUER-LOCK-VERBINDUNGEN**
DEVICE FOR SCREWING TOGETHER MEDIUM-GUIDING THREADED CONNECTIONS, PARTICULARLY LUER LOCK CONNECTIONS
DISPOSITIF POUR VISSER DES RACCORDS FILETES D'ELEMENTS ACHEMINANT UN FLUIDE, NOTAMMENT DES RACCORDS LUER-LOCK

(30) Priorität: 25.11.2002 CH 198802
(43) Veröffentlichungstag der Anmeldung: 31.08.2005
(73) Patentinhaber: TecPharma Licensing AG, 3401 Burgdorf (CH)
(72) Erfinder: LEHMANN, Hans-Ulrich, CH-3456 Trachselwald (CH)
(74) Vertreter: Detken, Andreas
(86) Internationale Anmeldenummer: PCT/CH2003/000759
(87) Internationale Veröffentlichungsnummer: WO 2004/047883

(56) Entgegenhaltungen:
- EP-A- 0 441 628
- WO-A-00/51667
- GB-A- 2 114 689
- US-A- 4 832 021
- US-A- 4 883 470
- US-A- 4 927 019
- US-A- 5 360 404
- US-A- 6 095 020

## Beschreibung

Die Erfindung betrifft eine Vorrichtung zum Verschrauben mediumführender Gewindeverbindungen, insbesondere Luer-Lock-Verbindungen, mit einem Aussenteil und einem darin drehbar aufgenommenen Innenteil, welcher über Mittel zur Aufnahme des einen, gewindetragenden Verbindungsteils oder eines mit diesem verdrehgesicherten Zusatzteils verfügt, wobei Hemmmittel vorhanden sind, welche eine Drehung des Innenteils gegenüber dem Aussenteil nur in eine Drehrichtung zulassen und dieser Drehung einen Widerstand entgegensetzen.

Luer-Lock Befestigungen werden insbesondere im Bereich der Medizintechnik eingesetzt, um Teile miteinander zu verbinden, in deren Innerem ein Medium, insbesondere eine Flüssigkeit fliesst. Zu diesen Teilen gehören beispielsweise Infusionsbehälter und zugehörige Leitungen oder Injektionsspritzen und zugehörige Nadeln. Damit solche Verbindungen dicht sind und sich nicht lösen, müssen sie mit einem gewissen Drehmoment angezogen werden. Andererseits dürfen die meistens aus Kunststoff bestehenden Gewindeteile auch nicht zu stark angezogen werden, weil sie sonst brechen können.

In der Druckschrift WO 00/51667 ist ein Aufbewahrungsbehälter für eine Injektionsspritze gezeigt, der in seiner Aussenseite eine Öffnung mit Längsrillen aufweist, in die eine Nadeleinheit mit in Längsrichtung verlaufenden Vorsprüngen eingesetzt werden kann. Die Nadeleinheit ist somit verdrehsicher in der Öffnung gehalten. Eine Injektionsvorrichtung kann nun zum Befestigen der Nadeleinheit auf die Nadeleinheit aufgesetzt und gegenüber dieser verdreht werden, bis die Nadeleinheit auf der Injektionsvomchtung festsitzt.

Der Erfindung liegt die Aufgabe zugrunde, eine Vorrichtung zum Anziehen flüssigkeitsführender Gewindeverbindungen, insbesondere Luer-Lock-Verbindungen vorzuschlagen, die einfach und kostengünstig herzustellen, einfach zu bedienen ist und das Drehmoment beim Anziehen von Gewindeverbindungen sowohl nach unten als auch nach oben sicher begrenzt.

Die Erfindung löst diese Aufgabe, indem der Widerstand beim Drehen bis zu einer Maximallast zunimmt und anschliessend stark abfällt. Der Widerstand verläuft also vorzugsweise sägezahnförmig, wobei die starke Abnahme des Drehmoments vom Benutzer der Vorrichtung taktil und/oder akustisch wahrgenommen werden kann, wodurch ihm signalisiert wir, dass das zum Anziehen der Gewindeverbindung gewünschte Drehmoment erreicht ist.

Nach einer bevorzugten Ausführungsart der Erfindung bestehen die Mittel zur Aufnahme aus einer im Innenteil vorhandenen Bohrung mit axial darin angeordneten Längsnuten. Diese Längsnuten nehmen die an einem üblichen Nadelträger des Luer-Lock-Typs oder an einem Zusatzteil vorhandenen Längsrippen auf und sorgen so für eine sichere Übertragung des Anzugs-Drehmoments. Der Zusatzteil ist bevorzugt eine auf den Verbindungsteil aufgesteckte Nadelschutzkappe.

Nach einer weiteren Ausführungsart der Erfindung weisen die Hemm-Mittel im Aussenteil angeordnete, im Wesentlichen axial ausgerichtete Vorsprünge auf, die mit am Innenteil angeordneten, im Wesentlichen axial ausgerichteten Vertiefungen zusammenwirken und es sind Federmittel vorhanden, weiche den Aussenteil und den Innenteil in axialer Richtung gegeneinander spannen. Diese Vorsprünge und Vertiefungen können in vorteilhafter Weise eine fühl- und sichtbare periodische axiale Verschiebung des Innenteils gegenüber dem Aussenteil bewirken. Selbstverständlich ist auch eine kinematische Umkehr dieses Prinzips möglich, bei der die Vertiefungen im Aussenteil und die Vorsprünge am Innenteil angeordnet sind.

Eine andere Ausführungsart der Erfindung sieht vor, dass die Hemm-Mittel im Aussenteil angeordnete, im Wesentlichen radial ausgerichtete Vorsprünge aufweisen, die mit am Innenteil angeordneten, im Wesentlichen tangential ausgerichteten Lamellen zusammenwirken, wobei die Lamellen in im Wesentlichen radialer Richtung federnd ausgebildet sind. Dies erlaubt eine besonders einfache Realisation der Vorrichtung mit nur zwei Teilen. Natürlich ist auch hier die kinematische Umkehr möglich, indem die Vorsprünge am Innenteil und die Lamellen im Aussenteil angeordnet werden.

Ein anderer Aspekt der Erfindung betrifft eine Ladestation für die Vorbereitung einer Injektionsvorrichtung, welche eine erfindungsgemässe Vorrichtung enthält. Dabei kann die Ladestationen noch andere funktionelle Elemente enthalten, die der Vorbereitung einer Injektionsvorrichtung, insbesondere eines Autoinjektors, dienen. Die erfindungsgemässe Vorrichtung ist vorzugsweise herausnehmbar in der Ladestation aufgenommen. Eine besondere Ausführungsart der Ladestation ist gleichzeitig als Behältnis zur Aufbewahrung von Teilen einer Injektionsvorrichtung ausgebildet. Dies ermöglicht dem Benutzer einer Injektionsvorrichtung, die benötigten Einzelteile einfach mit sich zu tragen und übersichtlich aufzubewahren.

Besondere Ausführungsarten der Erfindung werden nachfolgend unter Bezugnahme auf die beiliegenden Zeichnungen beispielsweise näher erläutert. Es zeigt:
- Figur 1: eine perspektivische Ansicht einer Injektionsnadel mit Luer-Lock- Verbindung,
- Figur 2: eine teilweise auseinander gezogene Ansicht von schräg oben auf eine erste Ausführungsart der erfindungsgemässen Vorrichtung,
- Figur 3: eine teilweise auseinander gezogene Ansicht von schräg unten auf die erste Ausführungsart gemäss Figur 2,
- Figur 4: einen Längsschnitt durch die erste Ausführungsart gemäss den Figu- ren 2 und 3,
- Figur 5: eine Ansicht von schräg unten auf eine zweite Ausführungsart der erfindungsgemässen Vorrichtung,
- Figur 6: einen Längsschnitt durch die zweite Ausführungsart gemäss Figur 5 und
- Figur 7: eine perspektivische Ansicht einer Ladestation für eine Injektionsvor- richtung, mit einer Vorrichtung zum Anziehen einer Gewindeverbin- dung.

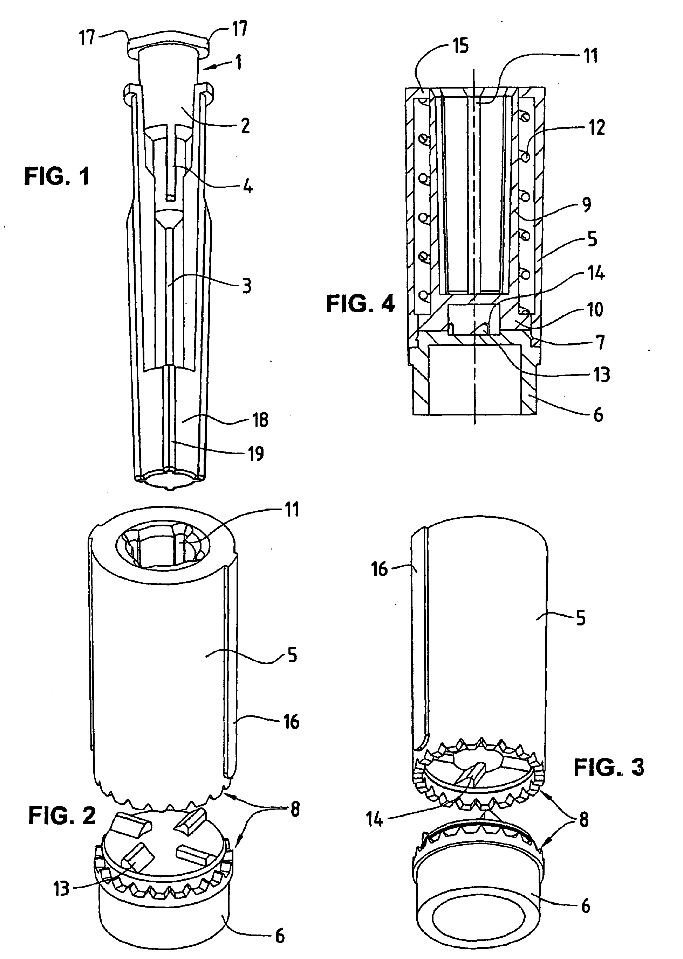

Die Figur 1 zeigt ein bekanntes Beispiel einer Injektionsnadel 1 mit einem Nadelträger 2 aus Kunststoff, in welchem eine Kanüle 3 aus Metall eingegossen ist. Der Nadelträger 2 weist an seinem in der Figur oberen Rand zwei Lappen 17 auf, welche die Funktion eines Aussengewindes übernehmen, das in ein entsprechendes Innengewinde eingeschraubt werden kann, um die injektionsnadel mit einer Spritze zu verbinden. Aussen am Nadelträger 2 sind Rippen 4 angeformt, die in entsprechenden Längsnuten in einer Nadelschutzkappe 18 aufgenommen sind und so mit dieser eine drehfeste Verbindung bilden. Die Nadelschutzkappe 18 weist ihrerseits äussere Rippen 19 auf, die ebenfalls der Übertragung eines Drehmoments dienen. Die im folgenden beschriebenen Ausführungsarten einer erfindungsgemässen Vorrichtung zum Anziehen flüssigkeitsführender Gewindeverbindungen sind speziell für Injektionsnadeln dieser Art bestimmt.

Die in den Figuren 2 bis 4 dargestellte erste Ausführungsart der Vorrichtung weist eine äussere Hülse 5 auf, die über eine Schnapp-Verbindung 7 mit einem Boden 6 verbunden ist. Damit sich die Hülse 5 gegenüber dem Boden 6 nicht drehen kann, sind beide Teile mit einer Verzahnung 8 versehen. In der Hülse 5 ist ein Drehteil 9 gelagert, der eine axiale Öffnung aufweist, in der Längsnuten 11 zur Aufnahme der Rippen 19 der Nadelschutzkappe 18 vorhanden sind. Zwischen der Hülse 5 und dem Drehteil 9 befindet sich eine als Druckfeder ausgebildete Feder 12, die sich axial an einem oberen Rand 15 der Hülse 5 und einem unteren Flansch 10 des Drehteils 9 abstützt und den Drehteil 9 gegen den Boden 6 drängt. Auf diesem Boden 6 sind eine Anzahl, im vorliegenden Beispiel vier Vorsprünge 13 angeordnet, von denen jeder eine vertikale und eine geneigte Flanke hat. In der unteren Stirnseite des Drehteils 9 sind Vertiefungen 14 eingearbeitet, die bezüglich Anzahl und Form den Vorsprüngen 13 entsprechen. Wenn nun beispielsweise eine Injektionsnadel 1 in den Drehteil 9 eingeführt wird, greifen die Rippen 4 am Nadelträger 2 in die Längsnuten 11 des Drehteils 9, so dass sich der Nadelträger 2 nicht im Drehteil 9 drehen kann. Wird nun die Spritze im Uhrzeigersinn gedreht, erfolgt zuerst eine relative Drehung zwischen der Spritze und dem Nadelträger 2, wobei das Gewinde angezogen wird. Wird ein vorbestimmtes minimales Drehmoment erreicht, beginnt sich der Drehteil 9 gegenüber der Hülse 5 zu drehen. Dabei gleiten die Vertiefungen 14 des Drehteils über die Vorsprünge 13 des Bodens 6, wobei der Drehteil entgegen der Kraft der Feder 12 so weit angehoben wird, bis - im vorliegenden Beispiel nach einem Drehwinkel von 90° - die Vertiefungen 14 wiederum mit den Vorsprüngen 13 fluchten. In diesem Moment wird der Drehteil 9 durch die Feder 12 nach unten gestossen und schlägt auf dem Boden 6 auf, wodurch dem Benutzer sowohl über das Gefühl als auch das Gehör signalisiert wird, dass das gewünschte Drehmoment erreicht ist. Am Aussenumfang der Hülse 5 vorgesehene Längsrippen 16 dienen einerseits dem besseren Halt, wenn die Vorrichtung mit der Hand gehalten wird und andererseits als Verdrehschutz, wenn die Vorrichtung in einer Ladestation angeordnet wird, wie dies weiter unten noch beschrieben wird.

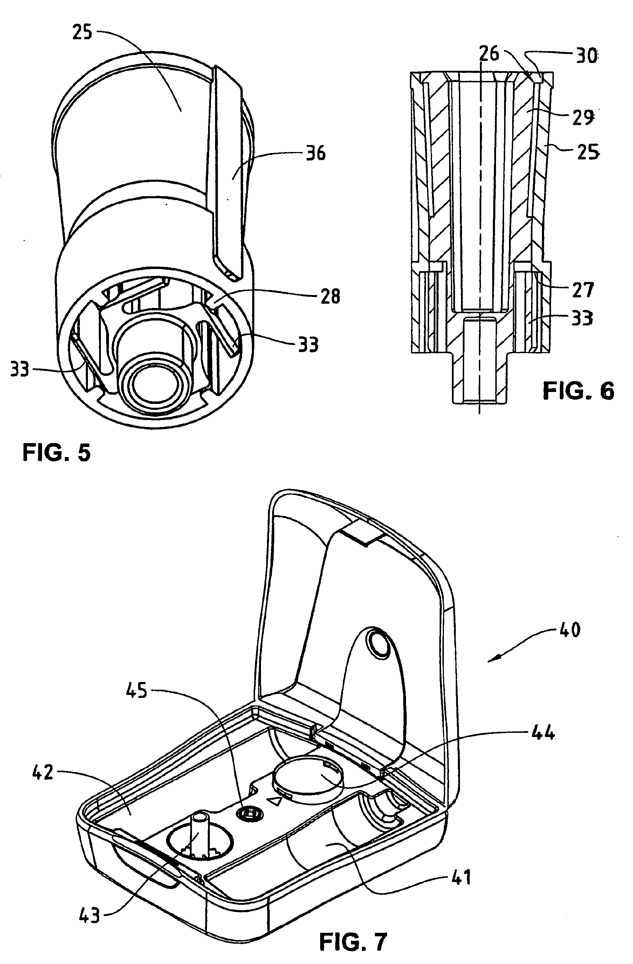

Die Figuren 5 und 6 zeigen eine zweite, vorteilhafte Ausführungsart der erfindungsgemässen Vorrichtung, welche aus nur zwei Teilen besteht, nämlich aus einer Hülse 25 und einem in der Hülse angeordneten Drehteil 29, die beide aus Kunststoff im Spritzgiessverfahren hergestellt werden können. Der Drehteil 29 weist in seinem unteren Teil tangential angeformte Lamellen 33 auf. Diese Lamellen 33 wirken als Biegefedern und werden beim Drehen des Drehteils 29 an Längsrippen 28 vorbei geführt, die innen in der Hülse 25 angeformt sind, wobei die Lamellen 33 radial federnd nachgeben. Nach dem Passieren der Längsrippen 28 schnappen die Lamellen 33 wieder nach aussen, wobei ein klickendes Geräusch entsteht, das dem Benutzer anzeigt, dass das gewünschte Drehmoment erreicht ist. Diese Vorrichtung ist sehr einfach zu montieren, indem der Drehteil 29 von oben in die Hülse 25 geschoben wird, wobei sich die Lamellen 33 im oberen, leicht konischen Teil der Hülse 25 symmetrisch nach innen verformen und nach dem Passieren des Absatzes 27 der Hülse 25 nach aussen schnappen. Dadurch ist der Drehteil nach oben gehalten. Nach unten ist der Drehteil 29 in der Hülse 25 durch einen am Drehteil 29 angeformten oberen Rand 30 gehalten, der an einem weiteren Absatz 26 der Hülse 25 ansteht. Auch bei diesem Ausführungsbeispiel sind am Aussenumfang der Hülse 25 Längsrippen 36 vorgesehen, die dem besseren Halt dienen, wenn die Vorrichtung mit der Hand gehalten wird, oder als Verdrehschutz dienen, wenn die Vorrichtung in einer Ladestation angeordnet wird, wie dies nachstehend beschrieben wird.

Aus der vorangehenden Beschreibung der beiden Ausführungsarten der Erfindung geht hervor, dass der Innenteil 9 beziehungsweise 29 nur in einer Richtung gedreht werden kann und in der Gegenrichtung gesperrt wird. Die erfindungsgemässe Vorrichtung kann daher auch zum Lösen von Gewindeverbindungen verwendet werden.

Figur 7 zeigt eine Ladestation 40 für die Vorbereitung eines Autoinjektors. Autoinjektoren dienen insbesondere zur Verabreichung von Medikamenten, die sich der betroffene Patient selbst injiziert. Sie arbeiten üblicherweise mit Federkraft, wobei automatisch zuerst eine Injektionsnadel eingestochen und danach ein Wirkstoff ausgeschüttet wird. Wenn der Autoinjektor zur Verwendung von gefüllten Spritzen ausgestattet ist, gehören das Spannen der Federn, das Aufsetzen der Injektionsnadel auf die Spritze und das Einsetzen der Spritze in den Autoinjektor zu diesen Vorbereitungen. Elemente, welche diese Vorbereitungen ermöglichen oder erleichtern, können beispielsweise einen Spannzapfen 43 umfassen, der zum Spannen der Federn dient, und es kann eine Aufnahme 44 für eine (nicht dargestellte) Montagehilfe vorgesehen sein, mit deren Hilfe der Autoinjektor nach dem Einsetzen der Spritze zusammengesetzt wird. Weiter kann die Ladestation 40 mit Aufnahmemulden 41 und 42 ausgestattet sein, die zur Aufbewahrung des Autoinjektors oder Teilen davon und/oder der erwähnten Montagehilfe dienen. Die vorliegende Ladestation enthält ferner eine Vorrichtung 45, wie sie vorangehend anhand zweier Ausführungsbeispiele beschrieben wurde und die zum Anziehen der Gewindeverbindung zwischen der Injektionsnadel und der Spritze dient. Diese Vorrichtung 45 ist vorzugsweise herausnehmbar in einer zylindrischen Ausnehmung der Ladestation aufgenommen, welche Ausnehmung Längsnuten aufweist, in welche die Längsrippen 16 beziehungsweise 36 der Vorrichtung passen.

## Patentansprüche

1. Vorrichtung zum Verschrauben einer flüssigkeitsführenden Gewindeverbindung zwischen einer Injektionsvorrichtung und einer Injektionsnadel (1), insbesondere Luer-Lock-Verbindung, mit einem Aussenteil (5; 25) und einem darin drehbar aufgenommenen Innenteil (9; 29), welcher über Mittel (11) zur Aufnahme eines gewindetragenden Verbindungsteils (2) der Injektionsnadel (1) oder eines mit dem gewindetragenden Verbindungsteil (2) der Injektionsnadel (1) verdrehgesicherten Zusatzteils (18) verfügt, **dadurch gekennzeichnet, dass** Hemmmittel (13, 14; 28, 33) vorhanden sind, welche eine Drehung des Innenteils (9; 29) gegenüber dem Aussenteil (5; 25) nur in eine Drehrichtung zulassen und dieser Drehung einen Widerstand entgegensetzen, der beim Drehen bis zu einer Maximallast zunimmt und anschliessend stark abfällt.

2. Vorrichtung nach Anspruch 1, **dadurch gekennzeichnet, dass** die Mittel zur Aufnahme auf einer im Innenteil (9; 29) vorhandenen Bohrung mit axial darin angeordneten Längsnuten (11) bestehen.

3. Vorrichtung nach Anspruch 1 oder 2, **dadurch gekennzeichnet, dass** die Hemmmittel (13, 14) im Aussenteil (5) angeordnete, im Wesentlichen axial ausgerichtete Vorsprünge (13) aufweisen, die mit am Innenteil (9) angeordneten, im Wesentlichen axial ausgerichteten Vertiefungen (14) zusammenwirken, wobei der Aussenteil (5) und der Innenteil (9) axial verschieblich zueinander angeordnet sind und Federmittel (12) vorhanden sind, welche den Aussenteil (5) und den Innenteil (9) in axialer Richtung gegeneinander spannen.

4. Vorrichtung nach Anspruch 1 oder 2, **dadurch gekennzeichnet, dass** die Hemmmittel (13, 14) im Aussenteil (5) angeordnete, im Wesentlichen axial ausgerichtete Vertiefungen aufweisen, die mit am Innenteil (9) angeordneten, im Wesentlichen axial ausgerichteten Vorsprünge zusammenwirken, wobei der Aussenteil (5) und der Innenteil (9) axial verschieblich zueinander angeordnet sind und Federmittel (12) vorhanden sind, welche den Aussenteil (5) und den Innenteil (9) in axialer Richtung gegeneinander spannen.

5. Vorrichtung nach Anspruch 1 oder 2, **dadurch gekennzeichnet, dass** die Hemmmittel (28, 33) im Aussenteil (25) angeordnete, im Wesentlichen radial ausgerichtete Vorsprünge (28) aufweisen die mit am Innenteil (29) angeordneten, im Wesentlichen tangential ausgerichteten Lamellen (33) zusammenwirken, wobei die Lamellen (33) in im Wesentlichen radialer Richtung federnd ausgebildet sind.

6. Vorrichtung nach Anspruch 1 oder 2, **dadurch gekennzeichnet, dass** die Hemmmittel (28, 33) am Innenteil (29) angeordnete, im Wesentlichen radial ausgerichtete Vorsprünge aufweisen, die mit am Aussenteil (25) angeordneten, im Wesentlichen tangential ausgerichteten Lamellen zusammenwirken, wobei die Lamellen in im Wesentlichen radialer Richtung federnd ausgebildet sind.

7. Vorrichtung nach einem der vorangehenden Ansprüche, **dadurch gekennzeichnet, dass** der Zusatzteil eine auf den Verbindungsteil (2) aufgesteckte Nadelschutzkappe (18) ist.

8. Ladestation für die Vorbereitung einer Injektionsvorrichtung, **dadurch gekennzeichnet, dass** sie eine Vorrichtung (45) nach einem der Ansprüche 1 bis 7 enthält.

9. Ladestation nach Anspruch 8, **dadurch gekennzeichnet, dass** die Vorrichtung (45) herausnehmbar in der Ladestation aufgenommen ist.

10. Ladestation nach Anspruch 8 oder 9, **dadurch gekennzeichnet, dass** sie als Behältnis zur Aufbewahrung von Teilen einer Injektionsvorrichtung ausgebildet ist.

## Claims

1. A device for screwing a liquid-carrying threaded connection between an injection device and an injection needle (1), in particular a Luer-Lock connection, having an outside part (5, 25) and an inside part (9; 29), which is rotatably accommodated in the former and has accommodating means (11) for receiving a thread-bearing connecting part (2) of the injection needle (1) or an additional part (18), which is secured in a twist-proof manner with the thread-bearing connecting part (2) of the injection needle (1), **characterized in that** inhibiting means (13, 14; 28, 33) are provided, allowing rotation of the inside part (9; 29) in only one direction of rotation with respect to the outside part (5; 25) and presenting a resistance to this rotation, this resistance increasing up to a maximum load during rotation and then dropping sharply.

2. The device according to claim 1, **characterized in that** the accommodating means consist of a borehole present in the inside part (9; 29) and having longitudinal grooves (11) arranged axially therein.

3. The device according to claim 1 or 2, **characterized in that** the inhibiting means (13, 14) have protrusions (13) that are arranged in the outside part (5) and are aligned essentially axially, cooperating with essentially axially aligned recesses (14) arranged on the inside part (9), whereby the outside part (5) and the inside part (9) are arranged to be axially displaceable in relation to one another, and spring means (12) are provided, tightening the outside part (5) and the inside part (9) against one another in the axial direction.

4. The device according to claim 1 or 2, **characterized in that** the inhibiting means (13, 14) have essentially axially aligned recesses in the outside part (5), said recesses cooperating with protrusions that are aligned essentially axially and arranged on the inside part (9), the outside part (5) and the inside part (9) being arranged to be axially displaceable in relation to one another, and spring means (12) being provided, mutually bracing the outside part (5) and the inside part (9) in the axial direction.

5. The device according to claim 1 or 2, **characterized in that** the inhibiting means (28, 33) have protrusions (28) aligned essentially radially and arranged in the outside part (25), cooperating with lamellae (33) aligned essentially tangentially and arranged on the inside part (29), the lamellae (33) being designed to be resilient in an essentially radial direction.

6. The device according to claim 1 or 2, **characterized in that** the inhibiting means (28, 33) have protrusions that are aligned essentially radially and are arranged on the inside part (29), cooperating with lamellae that are aligned essentially tangentially and are arranged on the outside part (5), the lamellae being designed to be resilient in an essentially radial direction.

7. The device according to any one of the preceding claims, **characterized in that** the additional part is a needle safety cap (18) placed on the connecting part (2).

8. A loading station for preparing an injection device, **characterized in that** it comprises a device (45) according to any one of claims 1 to 7.

9. The loading station according to claim 8, **characterized in that** the device (45) is held in the loading station in such a way that it can be removed.

10. The loading station according to claim 8 or 9, **characterized in that** it is designed as a container for storing parts of an injection device.

## Revendications

1. Dispositif de vissage d'un raccord fileté conducteur de liquide entre un dispositif d'injection et une aiguille d'injection (1), notamment raccord luer lock, comportant une pièce extérieure (5 ; 25) et une pièce intérieure (9 ; 29) reçue dedans de manière pivotable, laquelle pièce intérieure dispose de moyens (11) destinés à recevoir une pièce de raccordement porteuse de filetage (2) de l'aiguille d'injection (1) ou une pièce supplémentaire (18) bloquée de manière à empêcher sa rotation avec la pièce de raccordement porteuse de filetage (2) de l'aiguille d'injection (1), **caractérisé en ce qu'**il est prévu des moyens inhibiteurs (13, 14 ; 28, 33) qui ne permettent une rotation de la pièce intérieure (9 ; 29) par rapport à la pièce extérieure (5 ; 25) que dans un sens de rotation et opposent à cette rotation une résistance qui augmente en cas de rotation jusqu'à une charge maximale et diminue ensuite fortement.

2. Dispositif selon la revendication 1, **caractérisé en ce que** les moyens récepteurs consistent en un alésage pratiqué dans la pièce intérieure (9 ; 29) et pourvu de rainures longitudinales (11) disposées axialement dedans.

3. Dispositif selon la revendication 1 ou 2, **caractérisé en ce que** les moyens inhibiteurs (13, 14) présentent des saillies (13) disposées dans la pièce extérieure (5) et orientées sensiblement axialement qui coopèrent au niveau de renfoncements (14) disposés au niveau de la pièce intérieure (9) et orientés sensiblement axialement, la pièce extérieure (5) et la pièce intérieure (9) étant disposées de manière à pouvoir se déplacer axialement l'une par rapport à l'autre et étant prévus des moyens à ressort (12) qui serrent la pièce extérieure (5) et la pièce intérieure (9) l'une contre l'autre dans le sens axial.

4. Dispositif selon la revendication 1 ou 2, **caractérisé en ce que** les moyens inhibiteurs (13, 14) présentent des renfoncements orientés sensiblement axialement qui coopèrent au niveau de saillies disposées au niveau de la pièce intérieure (9) et orientées sensiblement axialement, la pièce extérieure (5) et la pièce intérieure (9) étant disposées de manière à pouvoir se déplacer axialement l'une par rapport à l'autre et étant prévus des moyens à ressort (12) qui serrent la pièce extérieure (5) et la pièce intérieure (9) l'une contre l'autre dans le sens axial.

5. Dispositif selon la revendication 1 ou 2, **caractérisé en ce que** les moyens inhibiteurs (28, 33) présentent des saillies (28) disposées au niveau de la pièce extérieure (25) et orientées sensiblement radialement, qui coopèrent au niveau de lamelles disposées au niveau de la pièce intérieure (29) et orientées sensiblement tangentiellement (33), les lamelles (33) étant de conformation élastiques dans le sens sensiblement radial.

6. Dispositif selon la revendication 1 ou 2, **caractérisé en ce que** les moyens inhibiteurs (28, 33) présentent des saillies disposées au niveau de la pièce intérieure (29) et orientées sensiblement radialement, qui coopèrent au niveau de lamelles disposées au niveau de la pièce extérieure (25) et orientées sensiblement tangentiellement (33), les lamelles étant de conformation élastiques dans le sens sensiblement radial.

7. Dispositif selon une des revendications précédentes, **caractérisé en ce que** la pièce supplémentaire est un capuchon protecteur d'aiguille (18) fiché sur la pièce de raccordement (2).

8. Station de chargement pour la préparation d'un dispositif d'injection, **caractérisé en ce qu'**il comprend un dispositif (45) selon une des revendications 1 à 7.

9. Dispositif selon la revendication 8, **caractérisé en ce que** le dispositif (45) est reçu dans la station de chargement de manière à pouvoir être sorti.

10. Dispositif selon la revendication 8 ou 9, **caractérisé en ce qu'**il se présente sous forme d'un réceptacle destiné à conserver des pièces d'un dispositif d'injection.
